# FASCICULE DE BREVET EUROPEEN

(11) **EP 0 724 411 B1**
(45) Date de publication et mention de la délivrance du brevet: **16.02.2000**
(21) Numéro de dépôt: 94931615.2
(22) Date de dépôt: 20.10.1994
(51) Int. Cl.: A61B 19/00

(54) **DISPOSITIF SUPPORT POUR INSTRUMENT MEDICAL OU CHIRURGICAL**
HALTER FÜR EIN MEDIZINISCHES ODER CHIRURGISCHES INSTRUMENT
HOLDER FOR A MEDICAL OR SURGICAL INSTRUMENT

(30) Priorité: 21.10.1993 FR 9312779
(43) Date de publication de la demande: 07.08.1996
(73) Titulaire: Ognier, Jean-François, 84500 Bollene Saint Pierre (FR)
(72) Inventeur: Ognier, Jean-François, 84500 Bollene Saint Pierre (FR)
(74) Mandataire: Maureau, Philippe
(86) Numéro de dépôt international: FR9401225
(87) Numéro de publication internationale: WO9510985

(56) Documents cités:
- EP-A- 0 167 345
- EP-A- 0 415 417
- EP-A- 0 486 999
- DE-A- 2 514 496
- FR-A- 2 339 936
- US-A- 4 867 404

## Description

De nombreuses interventions médicales ou chirurgicales nécessitent la mise en oeuvre de plusieurs instruments qui ne peuvent être manipulés par une seule personne. Tel est notamment le cas pour les interventions endoscopiques ou coelioscopiques.

Il est donc nécessaire de recourir à plusieurs opérateurs, même si la fonction remplie par certains opérateurs est très subalterne et consiste simplement dans le maintien, en une position déterminée, d'un instrument. Outre le fait que cette opération est peu intéressante à effectuer pour un assistant, la présence de celui-ci crée une gêne importante du fait de l'exiguïté des salles d'intervention médicales ou des blocs opératoires, c'est pourquoi il a été imaginé d'utiliser des appareils supports d'instruments.

Il existe actuellement des potences fixées sur la table d'opération et pouvant être réglées par des systèmes vis-écrous. Compte tenu des manipulations nécessaires des moyens de blocage, les réglages sont difficiles et longs, ce qui fait perdre tout intérêt à ce type d'appareil.

Il a été imaginé de mettre en oeuvre des dispositifs à bras flexible, par exemple à anneaux en friction les uns sur les autres (de type flexible de douche). Toutefois, de tels dispositifs ne permettent pas un positionnement précis et ne demeurent pas dans la position qui leur a été donnée lorsque l'on exerce un effort.

Les documents EP-A-0 167 345 et US-A-4 930 932 concernent chacun un dispositif comportant des moyens de fixation sur une table d'opération ou similaire reliés à des moyens supports d'un instrument médical ou chirurgical par un bras, dans lequel au moins un segment proximal du bras est constitué par une succession de rotules et d'éléments cylindriques servant de sièges aux rotules et disposés en alternance, des moyens de mise en compression axiale du bras étant prévus pour assurer, par pression, le blocage en rotation des rotules sur leurs sièges, ces moyens de mise en tension assurant également le blocage de l'instrument médical ou chirurgical dans son support et étant commandé depuis l'extrémité distale d'un segment distal démontable du bras.

Toutefois, un tel dispositif possède une rigidité moyenne en position de blocage et présente l'inconvénient de nécessiter un nombre important de rotules, car la traversée de chacune d'elles par le câble de blocage limite considérablement leur débattement angulaire.

Le document EP-A-O 415 417 révèle un rétracteur du type comportant des moyens de fixation sur une table d'opération ou similaire reliés à des moyens supports d'un instrument médical ou chirurgical par un bras, dans lequel au moins un segment proximal du bras est constitué par une succession de rotules et d'éléments cylindriques servant de sièges aux rotules et disposés en alternance, des moyens de mise en compression axiale du bras étant prévus pour assurer, par pression, le blocage en rotation des rotules sur leurs sièges, les moyens de mise en compression axiale du bras comprenant des moyens d'alimentation en fluide sous pression de tous les éléments cylindriques associés chacun à une rotule et les moyens de libération de la compression axiale du bras comportant un organe de manoeuvre, situé à l'extrémité distale du segment distal du bras, c'est-à-dire à proximité du support du porte-instrument.

Un but de l'invention est de fournir un dispositif support assurant une immobilisation parfaite de l'instrument après positionnement, y compris lorsque l'instrument est soumis à un effort extérieur. Un autre but est de permettre au praticien de réaliser un réglage et un blocage du dispositif de façon instantanée à l'aide d'une seule main.

Un autre but est de réaliser un dispositif possédant tous les degrés de liberté dans l'espace opératoire.

Un autre but est de permettre un démontage du support de l'instrument afin de réaliser la stérilisation de celui-ci.

Ces différents buts sont atteints par un dispositif support du type de celui révélé par le document EP 0 415 417 et dans lequel le bras comporte un segment rigide proximal et un segment rigide distal, le segment proximal ne comporte que deux rotules, une à chacune de ses extrémités, à savoir une rotule proximale, solidaire des moyens de fixation du bras à la table d'opération ou similaire et une rotule intermédiaire reliant le segment proximal au segment distal, le segment distal est démontable et porte à son extrémité distale une rotule distale servant de porte-instrument médical ou chirurgical, et les moyens de mise en compression axiale du bras assurent également le blocage de la rotule distale.

Suivant une forme d'exécution de l'invention, chaque rotule est associée à un siège constitué par une bague formant piston mobile dans un évidement, formant cylindre, de l'extrémité associée de l'élément cylindrique correspondant.

Avantageusement, les moyens de libération de la compression axiale du bras et de la rotule distale sont constitués par un clapet de mise à l'échappement du fluide sous pression et un organe de manoeuvre mécanique de ce clapet, situé à l'extrémité distale du segment distal du bras.

Suivant une forme d'exécution simple de l'invention, le fluide sous pression participant à la mise en compression axiale du bras est de l'air comprimé, chaque salle d'opération ou de soins étant normalement équipée d'une source d'air comprimé.

Dans ce cas, avantageusement, le siège, le plus proche de l'extrémité proximale du segment proximal du bras, de la rotule proximale est constitué par une bague formant piston, le cylindre ménagé à l'extrémité correspondante du premier élément cylindrique étant relié, en permanence, à une source d'air comprimé, tandis que le siège de la rotule intermédiaire, le plus éloigné de l'extrémité proximale du segment proximal du bras, est constitué par une bague formant piston, le cylindre ménagé à l'extrémité correspondante de l'élément cylindrique situé à l'extrémité distale du segment proximal du bras étant relié, par une canalisation centrale, au cylindre précité logeant la bague siège formant piston de la rotule proximale, ce même cylindre logeant un second piston ou piston auxiliaire monté en opposition avec le premier de manière à être constamment refoulé, par l'air comprimé, en direction de l'extrémité distale du segment distal du bras et à transmettre, à la bague siège formant piston de la rotule distale ou rotule porte-instrument, par l'intermédiaire d'une pièce tubulaire formant entretoise, la force axiale nécessaire au blocage de cette rotule distale.

Pour permettre au praticien de débloquer instantanément les trois rotules de ce bras sans avoir à interrompre l'alimentation en air comprimé à l'extrémité proximale du bras, selon une caractéristique intéressante de l'invention, il est prévu, d'une part, dans le piston auxiliaire, un canal axial équipé d'un clapet normalement maintenu en position de fermeture, par la pression de l'air comprimé et, d'autre part, à l'extrémité distale du segment distal du bras, c'est-à-dire au voisinage de la rotule porte-instrument, un levier de commande de mise à l'air libre de l'air comprimé, ce levier étant attelé à une tige mobile axialement, en direction du clapet précité, jusqu'à en commander l'ouverture, et faire, ainsi, communiquer le cylindre précité, qui loge ces deux pistons, avec l'intérieur d'une partie centrale tubulaire de ce segment distal du bras, partie centrale qui communique, avec l'extérieur, par des trous radiaux et des lumières axiales ménagés, à cet effet, dans ce segment distal du bras.

Enfin, suivant encore une autre caractéristique intéressante de l'invention évitant un déblocage intempestif de l'instrument médical ou chirurgical, dans sa rotule support, d'une part, à la bague siège formant piston et assurant normalement le blocage de cette rotule est associé un moyen à ressort agissant, sur cette bague siège, dans le même sens que l'air comprimé et, d'autre part, le siège de cette rotule distale opposé à celui sur lequel agit le piston auxiliaire, c'est-à-dire le siège situé à l'extrémité distale du segment distal du bras est constitué par l'extrémité interne d'une vis tubulaire vissée dans l'extrémité distale du segment distal du bras qui, à cet effet, est tubulaire et taraudée.

Ainsi, la commande de mise à l'air libre de l'air comprimé assurant le blocage des deux segments du bras ne libère pas totalement l'instrument médical ou chirurgical dans sa rotule porte-instrument. Ainsi, le praticien peut déplacer l'instrument par déformation du bras sans obligatoirement risquer de déplacer intempestivement l'instrument dans sa rotule porte-instrument. S'il veut procéder à un déplacement de l'instrument dans sa rotule, il peut le faire après avoir commandé la mise à l'air libre de l'air comprimé. Il lui suffit pour cela de dévisser la vis tubulaire qui constitue le siège de la rotule porte-instrument situé à l'extrémité distale du segment distal du bras.

Des essais ont démontré que ce dispositif support doit pouvoir résister à un effort, à l'extrémité distale du bras, de l'ordre de 50 N, effort qui s'ajoute au poids du bras et de l'instrument. La rotule proximale doit pouvoir s'opposer à un moment de 50 N.m. Cette opposition reposant sur la friction entre les rotules et leurs sièges, friction dont le rendement, compte tenu du coefficient de frottement métal/métal, est de l'ordre de 10 %, il est nécessaire de développer une force d'appui du siège mobile sur la rotule proximale égale à 10 000 N pour une rotule de 50 mm de diamètre, et par conséquent une pression de 51 X 10⁵ Pa.

Or, les pressions usuelles des réseaux de distribution d'air comprimé, en général inférieures à 8.10⁵ Pa, ne permettent pas de développer les efforts requis sans augmenter considérablement les dimensions du dispositifs.

Pour permettre de limiter ces dimensions à des valeurs raisonnables, suivant une forme d'exécution perfectionnée de l'invention, à chaque bague siège formant piston des rotules proximale et intermédiaire du bras, est associé au moins un piston secondaire, ayant un rôle multiplicateur d'efforts et agissant, sur la bague siège mobile considérée, par l'intermédiaire d'un second fluide, de préférence incompressible, tel que de l'huile hydraulique.

C'est ainsi que, dans l'extrémité, la plus éloignée de la rotule proximale, de la bague siège mobile de la rotule proximale est aménagé un cylindre secondaire non débouchant, destiné à loger la tête et une partie de la tige du piston secondaire associé à cette bague et dont l'extrémité distale forme la paroi proximale de la chambre de travail, alimentée en air comprimé, de cette bague, l'espace compris entre cette bague et le fond du cylindre qui la loge et qui entoure la tige de ce piston secondaire étant rempli de fluide secondaire.

Ainsi, dès que la chambre de travail, associée au siège mobile formant piston de la rotule proximale, est alimentée en air comprimé, le piston secondaire associé recule, mettant en pression le fluide secondaire dont l'action sur la bague siège mobile de la rotule proximale s'ajoute à celle de l'air comprimé sur cette bague.

Le piston auxiliaire possède une tige guidée dans un alésage axial d'un premier piston secondaire dont la face frontale constitue une partie annulaire du fond de la chambre de travail associé à la bague siège mobile de cette rotule intermédiaire et dont la tige est guidée dans un alésage axial d'une paroi frontale d'un second piston secondaire pourvue d'une jupe cylindrique dont le bord libre est en appui contre une face annulaire de la bague siège mobile de cette rotule intermédiaire, orientée dans la direction opposée à cette rotule, la chambre comprise entre la tête du piston auxiliaire et la face frontale du second piston secondaire et entourant la tige du piston auxiliaire étant remplie du fluide secondaire.

Ainsi, dès que la chambre de travail associée à la bague siège mobile de la rotule intermédiaire est alimentée en air comprimé, la tige du premier piston secondaire associé est refoulée dans la chambre remplie de fluide secondaire, située entre le piston auxiliaire et le second piston secondaire, mettant sous pression le fluide secondaire contenu dans cette chambre.

Il en résulte deux effets :
- une action de ce fluide secondaire contre le piston auxiliaire qui agit sur le siège mobile de la rotule distale, action qui s'ajoute à celle de l'air comprimé en la multipliant,
- une action de ce fluide secondaire contre le second piston secondaire qui agit sur la bague siège mobile de la rotule intermédiaire dans le même sens que l'air comprimé et qui s'ajoute à l'action de l'air comprimé, en la multipliant.

De toute façon, l'invention sera bien comprise à l'aide de la description qui suit, en référence au dessin schématique annexé représentant, à titre d'exemple non limitatif, une forme d'exécution de ce dispositif :
Figure 1 est une vue d'ensemble de ce dispositif, dans le cas où les moyens de mise en compression axiale du câble sont constitués par un câble tendeur ;
Figures 2 et 3 sont des vues, respectivement, de côté en élévation et en coupe axiale dans le plan de la figure 2, de l'extrémité proximale du bras de ce dispositif ;
Figures 4 et 5 sont des vues, respectivement, de côté en élévation et en coupe axiale dans le plan de la figure 4, de l'extrémité distale du bras de ce dispositif ;
Figure 6 est, à échelle agrandie, une vue partielle de figure 5 montrant le clapet de mise à l'air libre peu visible sur la figure 5.

Ce dispositif comporte des moyens 1 de fixation à une table d'opération 2, des moyens supports 3 d'un instrument médical ou chirurgical 4 qui est du type à rotules, la liaison entre les moyens de fixation à la table d'opération 2 et les moyens supports 3 de l'instrument 4 étant constitués par un bras flexible 105. Les moyens 1 de fixation du dispositif à la table d'opération 2 sont constitués par un étrier 6 pourvu d'une vis de serrage 7.

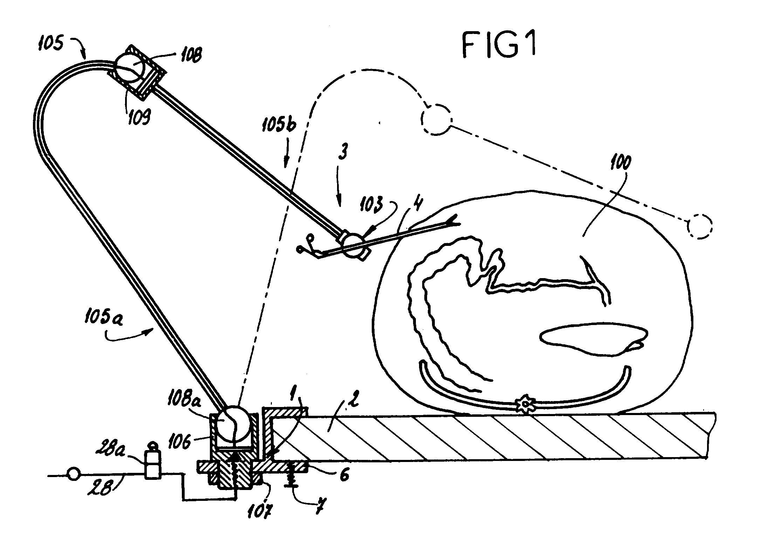

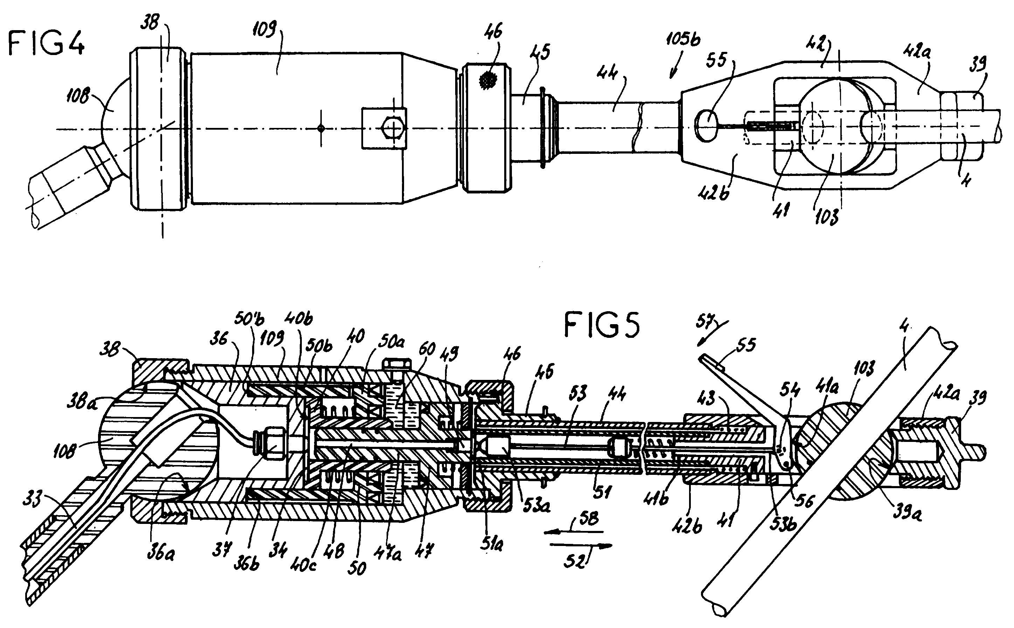

Les moyens 1 de fixation du bras 105 à la table d'opération 2 sont constitués par un bloc 106 pourvu d'une bride 107 solidaire de l'étrier 6, tandis que le support de l'instrument 4 est constitué par une rotule 103 prévue à l'extrémité distale du segment distal 105b du bras 105. Comme le montre la figure 1, le segment proximal 105a du bras 105 présente une première partie rectiligne rigide, solidaire d'une première rotule ou rotule proximale 108a, suivi d'une seconde partie curviligne dont l'extrémité libre est reliée, au segment distal 105b de ce bras 105, par une seule rotule intermédiaire 108. L'extrémité proximale du segment proximal 105a du bras 105 est reliée au bloc support 106 par la rotule proximale 108a. Comme le montre la figure 1, le déblocage des rotules 108a, 108 et 103 permet au praticien de déplacer l'instrument 4 entre les deux positions extrêmes montrées sur la figure 1, l'une à gauche de cette figure en traits épais et l'autre à droite de cette figure en traits mixtes.

Cette possibilité de manoeuvre du bras 105 d'une position extrême à l'autre ainsi que de l'instrument 4 dans sa rotule de support 103 permet au praticien d'aborder les organes sur lesquels il doit opérer et qui sont représentés succinctement en 100 sur cette figure, dans la position qu'il juge la plus commode.

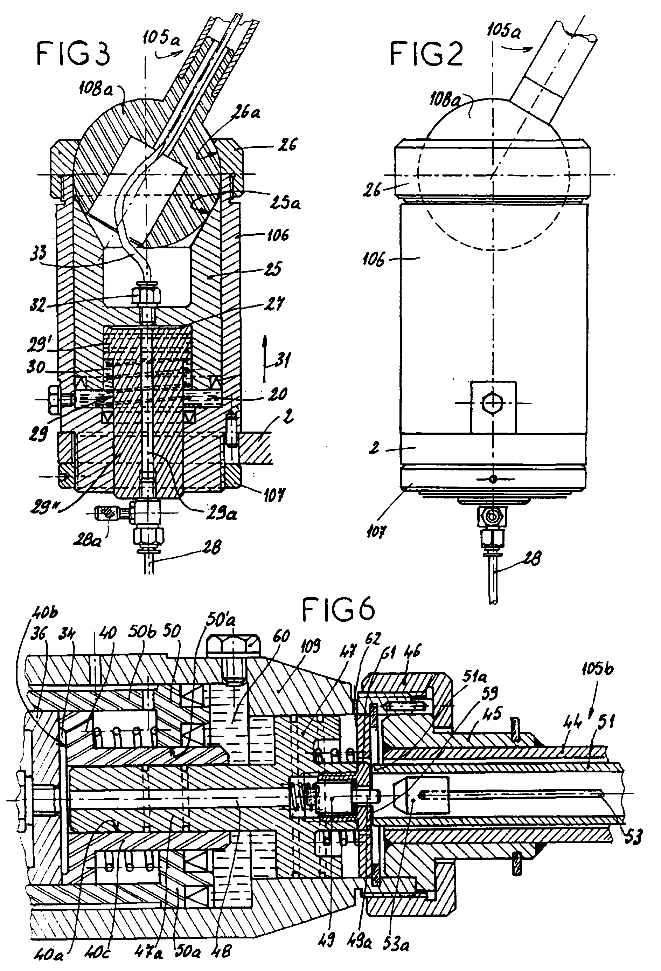

Les figures 2 et 3 montrent, à échelle agrandie, l'extrémité proximale du bras 105. Comme on peut le voir sur ces figures, le bloc support 106 de ce bras 105 constitue un cylindre dans lequel est montée, mobile axialement, une bague 25 dont l'extrémité distale est conformée en siège 25a pour la rotule proximale 108a. Une bague 26 vissée sur l'extrémité distale du bloc support 106 constitue le second siège 26a de la rotule 108a. Après montage, le siège 26a de la rotule 108a est donc considéré comme fixe tandis que son siège 25a formé dans la bague 25 constitue son siège mobile. La chambre de travail 27 du vérin constitué par le bloc support 106 et la bague 25 est située du côté proximal de cette dernière et est alimentée axialement, par une canalisation d'arrivée d'air comprimé 28 raccordée à un canal axial 29a traversant l'extrémité proximale du bloc support 106. Entre la canalisation 28 et le canal 29a est monté un gicleur 28a qui a pour fonction de limiter le débit d'air comprimé lors du déblocage des rotules, comme décrit ci-après, en vue de limiter la consommation en air comprimé lors de ce déblocage.

On conçoit aisément que l'alimentation en air comprimé de la chambre 27 provoque le déplacement de la bague 25 dans le sens de la flèche 31 et, par conséquent, le blocage de la rotule 108 entre ses deux sièges 25a et 26a.

Comme le montrent les figures 3 et 5, le canal 29a d'alimentation de la chambre 27 se prolonge, au-delà de cette dernière, par un raccord 32 et une canalisation souple 33 traversant la rotule proximale 108a et tout le segment proximal 105a du bras 105 ainsi que la rotule intermédiaire 108 jusqu'à la chambre de travail 34 d'un vérin constitué par un élément cylindrique 109 et une bague 36 dont l'extrémité proximale est conformée en siège 36a pour la rotule 108. Pour pénétrer dans la chambre de travail 34, la canalisation souple 33 traverse axialement la bague 36 au moyen d'un raccord 37. Comme le montre la figure 5, montrant plus particulièrement la rotule intermédiaire 108 et le segment distal 105b du bras 105, le second siège ou siège proximal 38a de la rotule 108 est constitué dans une bague filetée 38 vissée sur l'extrémité proximale, filetée à cet effet, de l'élément cylindrique 109. Ce siège 38a constitue donc le siège fixe de la rotule 108 tandis que son siège 36a en constitue le siège mobile permettant le blocage et le déblocage de cette rotule 108.

On conçoit donc que lorsque de l'air comprimé est introduit dans la chambre de travail 27 associée à la bague 25 dans laquelle est formé le siège 25a de la première rotule 108a, ce même air comprimé atteint la chambre de travail 34 du vérin associé à la bague 36 dans laquelle est formé le siège mobile 36a de la rotule intermédiaire 108. En conséquence, les deux rotules 108a et 108 peuvent donc être bloquées instantanément et simultanément.

Comme le montre plus particulièrement la figure 5, la rotule 103 ou rotule distale servant de support à l'instrument 4 est serrée entre une vis terminale 39 à l'extrémité proximale de laquelle est aménagé l'un des sièges ou siège fixe 39a de la rotule 103 et un siège 41a ou siège proximal aménagé à l'extrémité distale d'une bague formant piston 41 dont le montage et le rôle seront décrits ultérieurement.

La vis 39 est vissée dans un trou taraudé ménagé au centre de la traverse distale 42a de l'extrémité distale 42 du segment distal 105b du bras 105 conformée en cadre, à l'intérieur duquel est logée la rotule 103. Le piston 41 dans lequel est aménagé l'autre siège 41a de la rotule 103 est monté coulissant axialement à travers la seconde traverse ou traverse proximale 42b de cette extrémité en forme de cadre 42 du segment distal 105b du bras 105. Un ressort 43 assure l'application constante du siège 41a contre la rotule 103, après vissage de la vis 39 dans la traverse distale 42a du cadre 42.

Après montage de la vis 39, le siège proximal 41a constitue le siège mobile de la rotule 103, tandis que la siège 39a en constitue le siège fixe.

La traverse proximale 42b du cadre 42 est solidaire de l'extrémité distale d'un tube 44 dont l'extrémité proximale est solidaire d'un manchon épaulé 45 lui-même bridé à l'extrémité distale, filetée à cet effet, de l'élément cylindrique 109 au moyen d'une bague-écrou 46. Ce tube 44 constitue donc, avec le cadre 42, le segment distal 105b du bras 105.

Comme cela ressort de l'examen des figures 5 et 6, le segment distal 105b du bras 105 est donc facilement démontable de son segment proximal 105a, par simple dévissage de la bague 46.

Le fond de la chambre 34 associée à la bague 36 dans laquelle est formé le siège 36a de la rotule intermédiaire 108, est partiellement constitué par la tige 47a d'un piston auxiliaire 47 monté, axialement coulissant, dans l'extrémité distale de l'élément cylindrique 109. En outre, ce piston auxiliaire 47 et sa tige 47a sont traversés, axialement, par un canal 48 débouchant, du côté de son extrémité proximale, dans la chambre 34 et dont l'extrémité distale est fermée par un clapet 49 normalement maintenu en position de fermeture lorsque la chambre 34, et par conséquent le canal 48, sont alimentés en air comprimé, ce qui correspond à la position de blocage des rotules 108a, 108 et 103.

Par ailleurs, comme le montre la figure 5, l'extrémité proximale du piston 41 dans lequel est aménagé le siège 41a de la rotule 103 est conformée en tige 41b engagée dans l'extrémité distale d'une pièce tubulaire 51 dont l'extrémité proximale 51a est normalement en appui contre l'extrémité distale du piston auxiliaire 47. En conséquence, lorsque la chambre 34 est alimentée en air comprimé, le piston auxiliaire 47, qui constitue une partie de son fond, est déplacé axialement dans le sens de la flèche 52, entraînant, avec lui, la pièce tubulaire 51 et, par conséquent, le piston 41 dans lequel est aménagé le siège 41a de la rotule 103. L'alimentation en air comprimé de la chambre 34 provoque donc aussi le serrage se la rotule 103.

Pour permettre le déblocage simultané et instantané de l'ensemble des rotules 108a,108 et 103, il est prévu une tige 53 montée axialement mobile à l'intérieur de la pièce tubulaire 51, dont l'extrémité proximale 53a est située très près de la tige de manoeuvre 49a du clapet 49 tandis que son extrémité distale 53b est attelée, au moyen d'un axe 54, à un levier de commande 55 lui-même articulé sur un axe 56 porté par le piston 43. En conséquence, une manoeuvre du levier 55 dans le sens de la flèche 57 a pour effet de déplacer la tige de commande 53 dans le sens de la flèche 58, inverse de celui de la flèche 52 et, par conséquent, de manoeuvrer le clapet 49 dans le sens de son ouverture, ce qui a pour effet de faire communiquer le canal 48 avec l'air libre. En effet, l'air peut s'échapper au-delà du clapet 49, jusque dans l'alésage de la pièce tubulaire 51 et, de là, successivement, par des lumières radiales 59, axiales 61 et, de nouveau, radiales 62, sortir à l'extérieur de l'élément cylindrique 109. Comme indiqué précédemment, lors de la mise à l'air libre du canal 48 par manoeuvre du clapet 49 dans le sens de l'ouverture, le débit d'air est limité par le gicleur 28a.

Par conséquent, une simple manoeuvre du levier 55 par le praticien peut permettre à ce dernier de débloquer les rotules du bras 105 et de modifier la position de ce dernier jusqu'à obtention de la position idéale.

Cependant, pour éviter que le déblocage des trois rotules ne risque d'entraîner un déréglage de la position de l'instrument 4 dans la rotule 103 qui lui sert de support, il est prévu, dans l'évidement de la traverse proximale 42b qui sert de logement au piston 41, un ressort de rappel 43 qui tend constamment à pousser le piston 41 et, par conséquent, le siège 41a de la rotule 103, en direction de cette dernière. Le déblocage total de la rotule 103 pour permettre une modification de la position de l'instrument 4 dans cette rotule 103 ne peut donc être obtenu que par dévissage de la vis 39 à l'extrémité proximale de laquelle est aménagé le siège 39a de la rotule 103.

Comme indiqué précédemment, la pression des réseaux de distribution d'air comprimé est généralement de l'ordre de 8.10⁵ Pa. Or, il a été démontré que les efforts auxquels sont soumises les extrémités distales des dispositifs supports d'instruments médicaux ou chirurgicaux sont de l'ordre de 50 N, ce qui soumet la rotule proximale à un moment de l'ordre de 50 N.m. Or, la résistance ou couple de la rotule proximale, comme celle des autres rotules, repose sur la friction entre les rotules et leurs sièges, friction dont le rendement, compte tenu du coefficient de frottement métal/métal, est de l'ordre de 10 %. Il est donc nécessaire de développer une force d'appui du siège mobile sur la rotule proximale de l'ordre de 10 000 N pour une rotule de 50 mm de diamètre qui est considérée une dimension raisonnable. Or, cette force ne peut résulter que d'une pression de l'ordre de 51.10⁵ Pa. Par conséquent, pour permettre de limiter à des dimensions raisonnables les éléments constitutifs de ce dispositif support, il a été prévu, en association avec chaque bague siège formant piston des rotules proximale 108a et intermédiaire 108, au moins un piston secondaire agissant par l'intermédiaire d'un fluide secondaire sur la bague siège considérée et permettant de multiplier l'effort engendré par l'air comprimé sur cette bague siège. De préférence, le fluide secondaire est un fluide incompressible tel que de l'huile hydraulique.

C'est pourquoi, comme le montre plus particulièrement la figure 3 à l'occasion de la rotule proximale 108a, le fond de la chambre de travail 27 du vérin constitué par la bague piston 25 et le corps cylindrique 106 est constitué par la tête 29' d'un piston secondaire 29, traversé axialement par le canal 29a d'alimentation en air comprimé. Ce piston auxiliaire 29 est mobile dans un alésage cylindrique non débouchant 30 ménagé coaxialement à la bague 25, dans sa face proximale, c'est-à-dire la plus éloignée de la rotule proximale 108a. L'espace 20 compris entre la bague 25 et le fond du corps cylindrique 106, et qui entoure la tige 29'' de ce piston secondaire 29, constitue la chambre de travail associée à ce piston secondaire 29 et remplie du fluide secondaire qui, de préférence, est constitué par de l'huile hydraulique.

On conçoit aisément que, dès que la chambre de travail 27 associée à la bague siège 25 formant piston de la rotule proximale 108a est alimentée en air comprimé, le piston secondaire 29 qui lui est associé tend à reculer, c'est-à-dire à se déplacer dans le sens inverse de celui de la flèche 31, ce qui a pour effet de mettre en pression le fluide secondaire dont l'action sur la bague siège mobile 25 de la rotule proximale 108a s'ajoute à celle de l'air comprimé sur cette bague. Compte tenu de la dimension de la section transversale de la chambre 20 par rapport à celle de la chambre 27, l'effort engendré par le fluide secondaire sur la bague siège mobile 25 est bien supérieur à celui engendré par l'air comprimé. Dans l'exemple illustré sur le dessin, la force développée par la pression hydraulique est de l'ordre de 5 fois supérieure à celle développée par la pression pneumatique sur la bague 25.

L'examen des figures 5 et 6 montre qu'à la bague siège mobile 36 de la rotule intermédiaire 108 sont associés deux pistons secondaires coaxiaux 40 et 50. Comme le montrent les figures précitées, la tige 47a du piston auxiliaire 47 est guidée dans un alésage axial 40a du premier piston secondaire 40 dont la face frontale annulaire 40b constitue une partie du fond de la chambre de travail 34 associé à la bague siège mobile 36 de la rotule intermédiaire 108. La tige 40c de ce premier piston secondaire 40 est guidée dans un alésage axial 50'a d'une paroi frontale 50a du second piston secondaire 50. Cette paroi frontale 50a est pourvue d'une jupe cylindrique 50b dont le bord libre 50'b est en appui contre une face annulaire 36b de la bague siège mobile 36 de cette rotule intermédiaire 108, face annulaire 36b qui est orientée dans la direction opposée à cette rotule 108. La chambre 60 comprise entre la tête du piston auxiliaire 47 et la paroi frontale 50a du second piston auxiliaire 50 et qui entoure la tige 47a du piston auxiliaire 47 est remplie d'un fluide secondaire qui, de préférence, est incompressible tel que l'huile hydraulique.

Ainsi, dès que la chambre de travail 34 associée à la bague siège mobile 36 de la rotule intermédiaire 108 est alimentée en air comprimé, la tige 40c du premier piston secondaire 40 est refoulée dans la chambre 60, mettant en pression le fluide secondaire qu'elle contient. Il en résulte deux effets :
- le fluide secondaire sous pression de cette chambre 60 agit contre le piston auxiliaire 47 qui agit lui-même sur le siège mobile 41a de la rotule distale 103, cette action s'ajoutant à celle de l'air comprimé qui remplit la chambre de travail 34 ; dans la forme d'exécution représentée sur le dessin, compte tenu des dimensions relatives des éléments, la force développée par la pression hydraulique est de l'ordre de 9 fois supérieure à celle engendrée par la pression pneumatique ;
- la pression hydraulique régnant dans la chambre 60 agit aussi contre le second piston secondaire 50 en le pressant contre la bague siège mobile 36 de la rotule intermédiaire 108, dans le même sens que l'air comprimé dans la chambre 34, ces deux actions s'ajoutant ; dans l'exemple illustré sur le dessin, la force engendrée par la pression hydraulique dans la chambre 60 est de l'ordre de 9 fois supérieure à celle engendrée par l'air comprimé dans la chambre 34.

En conséquence, l'adjonction des pistons secondaires 29,40 et 50 a donc pour effet de permettre une augmentation considérable des efforts engendrés par l'air comprimé sans avoir à prévoir un réseau de distribution d'air comprimé d'une pression supérieure à celle dont on dispose usuellement.

Le dispositif vient d'être décrit dans le cas où le bras comprend deux tronçons seulement. Toutefois, le nombre de tronçons pourrait être plus élevé, sans que l'on sorte du cadre de l'invention.

## Revendications

1. Dispositif support pour instrument médical ou chirurgical, du type comportant des moyens (1) de fixation sur une table d'opération (2) ou similaire reliés à des moyens (3) supports d'un instrument médical ou chirurgical (4) par un bras (5,105), dans lequel au moins un segment proximal (5a,105a) du bras (5,105) est constitué par une succession de rotules (8,108a,108) et d'éléments cylindriques (9,106,109) servant de sièges aux rotules et disposés en alternance, des moyens (15,27,34) de mise en compression axiale du bras (5,105) étant prévus pour assurer, par pression, le blocage en rotation des rotules (8,108a,108) sur leurs sièges (9,25a,26a,36a,38a), les moyens de mise en compression axiale du bras comprenant des moyens d'alimentation (28,29,33) en fluide sous pression de tous les éléments cylindriques associés chacun à une rotule (108a,108,103) et les moyens de libération de la compression axiale du bras (105) comportant un organe de manoeuvre (53,55), situé à l'extrémité distale du segment distal (105b) du bras (105), c'est-à-dire à proximité du support du porte-instrument (103),
caractérisé en ce que le bras comporte un segment rigide proximal (105a) et un segment distal (105b),
en ce que le segment proximal (105a) ne comporte que deux rotules, une à chacune de ses extrémités, à savoir une rotule proximale (108a), solidaire des moyens de fixation (1) du bras (105) à la table d'opération (2) ou similaire et une rotule intermédiaire reliant le segment proximal au segment distal (105b),
en ce que le segment distal est démontable et porte à son extrémité distale une rotule distale (103) servant de porte-instrument médical ou chirurgical,
en ce que les moyens de mise en compression axiale du bras assurent également le serrage de la rotule distale (103), et
en ce que les moyens de libération de la compression axiale du bras (105) et de la rotule distale (103) sont constitués par un clapet (49) de mise à l'échappement du fluide sous pression et un organe de manoeuvre (53, 55) mécanique de ce clapet.

2. Dispositif selon la revendication 1, caractérisé en ce que chaque rotule (108a, 108, 103) est associée à un siège constitué par une bague formant piston mobile (25, 36, 41) dans un évidement, formant cylindre, de l'extrémité associée de l'élément cylindrique correspondant (106, 109, 42b).

3. Dispositif selon la revendication 1 ou 2, caractérisé en ce que l'organe de manoeuvre (53,55) mécanique de ce clapet est situé à l'extrémité distale du segment distal (105b) du bras (105).

4. Dispositif selon l'une quelconque des revendications 1 à 3, caractérisé en ce que le fluide sous pression participant à la mise en compression axiale du bras (105) est de l'air comprimé.

5. Dispositif selon la revendication 3 ou 4, caractérisé en ce que le siège (25a), le plus proche de l'extrémité proximale du segment proximal (105a) du bras (105), de la rotule proximale (108a) est constitué par une bague formant piston (25), le cylindre ménagé à l'extrémité correspondante du premier élément cylindrique (106) étant relié, en permanence, à une source d'air comprimé, tandis que le siège (36a) de la rotule intermédiaire (108), le plus éloigné de l'extrémité proximale du segment proximal (105a) du bras (105), est constitué par une bague (36) formant piston, le cylindre ménagé à l'extrémité correspondante de l'élément culindrique (109) situé à l'extrémité distale du segment proximal (105a) du bras (105) étant relié, par une canalisation centrale (33,37), au cylindre précité (106) logeant la bague siège (25a) formant piston de la rotule proximale (108a), ce même cylindre (109) logeant un second piston (47) ou piston auxiliaire monté en opposition avec le premier (36) de manière à être constamment refoulé, par l'air comprimé, en direction de l'extrémité distale du segment distal (105b) du bras (105) et à transmettre, à la bague siège (41,41a) formant piston de la rotule distale (103) ou rotule porte-instrument, par l'intermédiaire d'une pièce tubulaire (51) formant entretoise, la force axiale nécessaire au blocage de cette rotule distale (103).

6. Dispositif selon la revendication 5, caractérisé en ce qu'il est prévu d'une part, dans le piston auxiliaire (47), un canal axial (48) équipé du clapet (49) normalement maintenu en position de fermeture, par la pression de l'air comprimé et, d'autre part, à l'extrémité distale du segment distal (105b) du bras (105), c'est-à-dire au voisinage de la rotule porte-instrument (103), un levier (55) de commande de mise à l'air libre de l'air comprimé, ce levier (55) étant attelé à une tige (53) mobile axialement, en direction du clapet précité (49), jusqu'à en commander l'ouverture et faire, ainsi, communiquer le cylindre précité (109), qui loge ces pistons (36,47), avec l'intérieur d'une partie centrale tubulaire de ce segment distal (105b) du bras (105), partie centrale qui communique, avec l'extérieur, par des troux radiaux (59,62) et des lumières axiales (61) ménagés, à cet effet, dans ce segment distal (105b) du bras (105).

7. Dispositif selon l'une quelconque des revendications 2 à 6, caractérisé en ce que, d'une part, à la bague siège (41,41a) formant piston et assurant normalement le blocage de la rotule distale (103) est associé un moyen à ressort (43) agissant, sur cette bague siège (41,41a), dans le même sens que l'air comprimé et, d'autre part, le siège (39a) de cette rotule distale (103) opposé à celui (41a) sur lequel agit le piston auxiliaire (47), c'est-à-dire le siège situé à l'extrémité distale du segment distal (105b) du bras (105) est constitué par l'extrémité interne d'une vis tubulaire (39) vissée dans l'extrémité distale du segment distal (105b) du bras (105) qui, en effet, est tubulaire et taraudée.

8. Dispositif selon l'une quelconque des revendications 1 à 7, caractérisé en ce qu'un gicleur (28a) limiteur de débit est monté sur la canalisation (28) d'alimentation en air comprimé.

9. Dispositif delon l'une quelconque des revendications 2 à 8, caractérisé en ce qu'à chaque bague siège formant piston (25,36) des rotules proximale (108a) et intermédiaire (108) du bras (105), est associé au moins un piston secondaire (29,40,50), ayant un rôle multiplicateur d'efforts et agissant, sur la bague siège mobile considérée, par l'intermédiaire d'un second fluide, de préférence incompressible.

10. Dispositif selon la revendication 9, caractérisé en ce que, dans l'extrémité, la plus éloignée de la rotule proximale (108a), de la bague siège mobile (25) de la rotule proximale (108a) est aménagé un cylindre secondaire (30) non débouchant, destiné à loger la tête (29') et une partie de la tige (29'') du piston secondaire (29) associé à cette bague (25) et dont l'extrémité distale forme la paroi proximale de la chambre de travail (27), alimentée en air comprimé, de cette bague (25), l'espace (20) compris entre cette bague (25) et le fond du cylindre (106) qui la loge et qui entoure la tige (29'') de ce piston secondaire (29) étant rempli de fluide secondaire.

11. Dispositif selon l'une des revendications 9 et 10, caractérisé en ce que le piston auxilaire (47) associé à la rotule intermédiaire (108) possède une tige (47a) guidée dans un alésage axial (40a) d'un premier piston secondaire (40) dont la face frontale (40b) constitue une partie annulaire du fond de la chambre de travail (34) associé à la bague siège mobile (36) de cette rotule intermédiaire (108) et dont la tige (40c) est guidée dans un alésage axial (50'a) d'une paroi drontale (50a) d'un second piston secondaire (50) pourvue d'une jupe cylindrique (50b) dont le bord libre (50'b) est en appui contre une face annulaire (36b) de la bague siège mobile (36) de cette rotule intermédiaire (108) orientée dans la direction opposée à cette rotule, la chambre (60) comprise entre la tête du piston auxilaire (47) et la face frontale (50a) du second piston secondaire (50) et entourant la tige (47a) du piston auxiliaire (47) étant remplie du fluide secondaire.

12. Dispositif selon l'une quelconque des revendications 9 à 11, caractérisé en ce que le fluide secondaire est de l'huile hydraulique.

## Claims

1. Support device for a medical or surgical instrument, of the type which comprises means (1) for securing to an operating table (2) or the like, which are connected to means (3) for supporting a medical or surgical instrument (4) by means of an arm (5,105), in which at least one proximal segment (5a,105a) of the arm (5,105) consists of a succession of ball joints (8,108a,108) and cylindrical components (9,106,109) which act as seats for the ball joints, and are disposed alternately, means (15,27,34) for axial compression of the arm (5,105) being provided in order to assure locking in rotation of the ball joints (8,108a,108) on their seats (9,25a,26a,36a,38a) by means of pressure, the means for axial compression of the arm comprising means (28,29,33) for supplying pressurised fluid to all the cylindrical components, each of which is associated with a ball joint (108a,108,103), and means for releasing the axial compression of the arm (105), comprising a manoeuvring unit (53,55), which is disposed at the distal end of the distal segment (105b) of the arm (105), i.e. in the vicinity of the instrument-holder support (103),
characterised in that the arm comprises a rigid proximal segment (105a) and a distal segment (105b);
in that the proximal segment (105a) comprises only two ball joints, one at each of its ends, i.e. a proximal ball joint (108a), which is integral with the means (1) for securing the arm (105) to the operating table (2) or the like, and an intermediate ball joint, which connects the proximal segment to the distal segment (105d);
in that the distal segment can be dismantled, and supports at its distal end a distal ball joint (103), which acts as a medical or surgical instrument-holder;
in that the means for axial compression of the arm also clamp the distal ball joint (103); and
in that the means for releasing the axial compression of the arm (105) and the distal ball joint (103) consist of a flap valve (49), for release of the pressurised fluid, and a unit (53,55) for mechanical manoeuvring of this flap valve.

2. Device according to claim 1, characterised in that each ball joint (108a,108,103) is associated with a seat constituted by a ring which forms a mobile piston (25,36,41) in a recess, which forms a cylinder, of the associated end of the corresponding cylindrical component (106,109,42b).

3. Device according to claim 1 or claim 2, characterised in that the unit (53,55) for mechanical manoeuvring of this flap valve is disposed at the distal end of the distal segment (105b) of the arm (105).

4. Device according to any one of claims 1 to 3, characterised in that the pressurised fluid which participates in axial compression of the arm (105) is compressed air.

5. Device according to claim 3 or claim 4, characterised in that the seat (25a) which is closest to the proximal end of the proximal segment (105a) of the arm (105) of the proximal ball joint (108a) consists of a ring which forms a piston (25), the cylinder which is provided at the corresponding end of the first cylindrical component (106) being permanently connected to a source of compressed air, whereas the seat (36a) of the intermediate ball joint (108) which is furthest away from the proximal end of the proximal segment (105a) of the arm (105), consists of a ring (36) which forms a piston, the cylinder which is provided at the corresponding end of the cylindrical component (109) disposed at the distal end of the proximal segment (105a) of the arm (105) being connected, by means of a central duct (33,37), to the aforementioned cylinder (106), which accommodates the seat ring (25a) which forms a piston of the proximal ball joint (108a), this same cylinder (109) accommodating a second piston (47) or auxiliary piston which is mounted in opposition with the first cylinder (36), such as to be continuously thrust rearward by the compressed air, in the direction of the distal end of the distal segment (105b) of the arm (105), and to transmit to the seat ring (41,41a) which forms the piston of the distal ball joint (103) or instrument-holder ball joint, by means of a tubular part (51) which forms a brace, the axial force which is necessary for locking of this distal ball joint (103).

6. Device according to claim 5, characterised in that there is provided firstly in the auxiliary piston (47) an axial duct (48) which is provided with the flap valve (49), which is normally maintained in the closure position, by the pressure of the compressed air, and secondly, at the distal end of the distal segment (105b) of the arm (105), i.e. in the vicinity of the instrument-holder ball joint (103), a control lever (55), for release to the open air of the compressed air, this lever (55) being connected to a rod (53) which is mobile axially, in the direction of the aforementioned flap valve (49), until it opens the latter, and thus makes the aforementioned cylinder (109), which accommodates these pistons (36,47), communicate with the interior of a central tubular part of this distal segment (105b) of the arm (105), which central part communicates with the exterior via radial holes (59,62) and axial apertures (61) which are provided for this purpose in this distal segment (105b) of the arm (105).

7. Device according to any one of claims 2 to 6, characterised in that, firstly, with the seat ring (41, 41a) which forms a piston, and normally provides locking of the distal ball joint (103), there is associated a spring means (43), which acts on this seat ring (41, 41a) in the same direction as the compressed air, and secondly, the seat (39a) of this distal ball joint (103) which is opposite that (41a) on which the auxiliary piston (47) acts, i.e. the seat which is disposed at the distal end of the distal segment (105b) of the arm (105), consists of the inner end of a tubular screw (39), which is screwed into the distal end of the distal segment (105b) of the arm (105), which, in fact, is tubular and threaded.

8. Device according to any one of claims 1 to 7, characterised in that a flow-limiting nozzle (28a) is mounted on the compressed air supply duct (28).

9. Device according to any one of claims 2 to 8, characterised in that, with each seat ring which forms a piston (25,36) of the proximal ball joint (108a) and intermediary ball joint (108) of the arm (105), there is associated at least one secondary piston (29,40,50) which plays a force-boosting role, and acts on the mobile seat ring in question by means of a second fluid, which preferably cannot be compressed.

10. Device according to claim 9, characterised in that, in the furthest end of the proximal ball joint (108a) of the mobile seat ring (25) of the proximal ball joint (108a), there is provided a secondary cylinder (30) which does not have an outlet, which is designed to accommodate the head (29') and part of the rod (29'') of the secondary piston (29) which is associated with this ring (25), and the distal end of which forms the proximal wall of the work chamber (27), which is supplied with compressed air, of this ring (25), the space (20) between this ring (25) and the base of the cylinder (106) which accommodates it, and which surrounds the rod (29'') of this secondary piston (29) being filled with secondary fluid.

11. Device according to one of claims 9 and 10, characterised in that the auxiliary piston (47) which is associated with the intermediate ball joint (108) has a rod (47a), which is guided in an axial bore (40a) of a first secondary piston (40), the front surface (40b) of which constitutes an annular part of the base of the work chamber (34), which is associated with the mobile seat ring (36) of this intermediate ball joint (108), and the rod (40c) of which is guided in an axial bore (50'a) of a front wall (50a) of a second secondary piston (50), which is provided with a cylindrical skirt (50b), the free edge (50'b) of which is supported against an annular surface (36b) of the mobile seat ring (36) of this intermediate ball joint (108), which is oriented in the direction opposite this ball joint, the chamber (60) which is between the head of the auxiliary piston (47) and the front surface (50a) of the second secondary piston (50), and surrounds the rod (47a) of the auxiliary piston (47) being filled with the secondary fluid.

12. Device according to any one of claims 9 to 11, characterised in that the secondary fluid is hydraulic oil.

## Patentansprüche

1. Haltevorrichtung für ein medizinisches oder chirurgisches Instrument, umfassend Mittel (1) zur Befestigung an einem Operationstische (2) o.ä., die mit Mitteln (3) zum Halten eines medizinischen oder chirurgischen Elements (4) verbunden sind, und zwar durch einen Arm (5, 105) in dem zumindest ein proximales Segment (5a, 105e) des Arms (5, 105) durch eine Aufeinanderfolge von Gelenkköpfen (8, 108a, 108) und zylindrischen Elementen (9, 106, 109), die den Gelenkköpfen als Sitz dienen und abwechselnd angeordnet sind, gebildet wird, wobei Mittel (15, 27, 34) zur axialen Unterdrucksetzung des Arms (5, 105) vorgesehen sind, um durch Druck die Rotationsbewegung der Gelenkköpfe (8, 108a, 108) in ihren Sitzen (9, 25a, 26a, 36a, 38a) zu blockieren, wobei die Mittel zur axialen Unterdrucksetzung des Arms Mittel (28, 29, 33) umfassen, um alle zylindrischen Elemente, die jeweils mit einem Gelenkkopf (108a, 108, 103) verbunden sind, mit einem unter Druck befindlichen Fluid zu speisen, und wobei die Mittel zur Freigabe der axialen Unterdrucksetzung des Arms (105) ein Bedienungsorgan (53, 55) umfassen, das am distalen Ende des distalen Segments (105b) des Arms (105), d.h. in der Nähe der Instrumentenhalterauflage (103), angeordnet ist,
dadurch gekennzeichnet, daß der Arm ein steifes proximales Segment (105a) und ein distales Segment (105b) umfaßt,
daß das proximale Segment (105a) nur zwei Gelenkköpfe umfaßt, und zwar einen an jedem seiner Enden, nämlich einen proximalen Gelenkkopf (108a), der mit den Mitteln (1) zur Befestigung des Arms (105) am Operationstisch (2) o.ä. verbunden ist, und einen Zwischen-Gelenkkopf, der das proximale Segment mit dem distalen Segment (105b) verbindet,
daß das distale Segment demontierbar ist und an seinem distalen Ende einen distalen Gelenkkopf (103) trägt, der als medizinischer oder chirurgischer Instrumententräger dient,
daß die Mittel zur axialen Unterdrucksetzung des Arms auch für das Festklemmen des distalen Gelenkkopfes (103) sorgen, und
daß die Mittel zur Freigabe der axialen Unterdrucksetzung des Arms (15) und des distalen Gelenkkopfes (103) durch ein Ventil (49) zum Auslassen des unter Druck befindlichen Fluids und ein mechanisches Organ (53, 55) zur Bedienung dieses Ventils gebildet werden.

2. Vorrichtung nach Anspruch 1, dadurch gekennzeichnet, daß jeder Gelenkkopf (108a, 108, 103) mit einem Sitz verbunden ist, der durch einen Ring gebildet wird, der einen beweglichen Kolben (25, 36, 41) in einer zylinderförmigen Aussparung des beteiligten Endes des entsprechenden zylindrischen Elements (106, 109, 42b) bildet.

3. Vorrichtung nach Anspruch 1 oder 2, dadurch gekennzeichnet, daß das mechanische Organ (53, 55) zur Bedienung dieses Ventils am distalen Ende des distalen Segments (105b) des Arms (105) angeordnet ist.

4. Vorrichtung nach einem der Ansprüche 1 bis 3, dadurch gekennzeichnet, daß das unter Druck befindliche Fluid, das an der axialen Unterdrucksetzung des Arms (105) beteiligt ist, Druckluft ist.

5. Vorrichtung nach Anspruch 3 oder 4, dadurch gekennzeichnet, daß der sich am nächsten zum proximalen Ende des proximalen Segments (105a) des Arms (105) befindliche Sitz (25a) des proximalen Gelenkkopfes (108a) durch einen Ring (25) gebildet wird, der einen Kolben bildet, wobei der Zylinder, der am entsprechenden Ende des ersten zylindrischen Elements (106) gebildet ist, permanent mit einer Druckluftquelle verbunden ist, während der sich am weitesten vom proximalen Ende des proximalen Segments (105a) des Arms (105) entfernt befindliche Sitz (36a) des Zwischen-Gelenkkopfes (108) durch einen Ring (36) gebildet wird, der einen Kolben bildet, wobei der Zylinder, der am entsprechenden Ende des zylindrischen Elements (109) am distalen Ende des proximalen Segments (105a) des Arms (105) gebildet ist, durch eine zentrale Leitung (33, 37) mit dem obengenannten Zylinder (106) verbunden ist, der den einen Kolben bildenden Ring-Sitz (25a) des proximalen Gelenkkopfes (108a) aufnimmt, wobei dieser Zylinder (109) einen zweiten Kolben (47) oder Hilfskolben aufnimmt, der gegenüberliegend vom ersten (36) montiert ist, so daß er ständig durch die Druckluft in Richtung auf das distale Ende des distalen Segments (105b) des Arms (105) zurückgedrängt wird, und so daß er auf den einen Kolben bildenden Ring-Sitz (41, 41a) des distalen Gelenkkopfes (103) oder Instrumentenhalter-Gelenkkopfes über ein rohrförmiges, eine Verbindung bildendes Teil (51) die axiale Kraft überträgt, die zur Blockierung dieses distalen Gelenkkopfes (103) notwendig ist.

6. Vorrichtung nach Anspruch 5, dadurch gekennzeichnet, daß folgendes vorgesehen ist: einerseits im Hilfskolben (47) ein axialer Kanal (48), der mit dem Ventil (49) ausgestattet ist, das durch den Druck der Druckluft normalerweise in geschlossener Position gehalten wird, und andererseits am distalen Ende des distalen Segments (105b) des Arms (105), d.h. in der Nähe des Instrumentenhalter-Gelenkkopfes (103) ein Betätigungshebel (55), um die Druckluft an die freie Luft abzugeben, wobei dieser Hebel (55) mit einem Stab (53) gekoppelt ist, der axial in Richtung auf das obengenannte Ventil (49) beweglich ist, bis er dessen Öffnung steuert und auf diese Weise den obengenannten Zylinder (109), der diese Kolben (36, 47) aufnimmt, mit dem Inneren eines zentralen rohrförmigen Teils dieses distalen Segments (105b) des Arms (105) verbindet, wobei dieser zentrale Teil über radiale Löcher (59, 63) und axiale Öffnungen (61), die zu diesem Zweck in diesem distalen Segment (105b) des Arms (105) gebildet sind, mit außen verbunden ist.

7. Vorrichtung nach einem der Ansprüche 2 bis 6, dadurch gekennzeichnet, daß einerseits mit dem Ring-Sitz (41, 41a), der einen Kolben bildet und normalerweise für die Blockierung des distalen Gelenkkopfes (103) sorgt, ein Federmittel (43) verbunden ist, das auf diesen Ring-Sitz (41, 41a) in der gleichen Richtung wie die Druckluft einwirkt, und andererseits der Sitz (39a) dieses distalen Gelenkkopfes (103), der dem Sitz (41a), auf den der Hilfskolben (47) einwirkt, gegenüberliegt, d.h. der Sitz, der sich am distalen Ende des distalen Segments (105b) des Arms (105) befindet, durch das innere Ende einer rohrförmigen Schraube (39) gebildet wird, die in das distale Ende des distalen Segments (105b) des Arms (105) geschraubt wird, das rohrförmig und mit einem Innengewinde versehen ist.

8. Vorrichtung nach einem der Ansprüche 1 bis 7, dadurch gekennzeichnet, daß eine durchsatzbegrenzende Düse (28a) an der Leitung (28) zur Speisung mit Druckluft montiert ist.

9. Vorrichtung nach einem der Ansprüche 2 bis 8, dadurch gekennzeichnet, daß mit jedem einen Kolben (25, 36) bildenden Ring-Sitz des proximalen Gelenkkopfes (108a) und des Zwischen-Gelenkkopfes (108) des Arms (105) zumindest ein sekundärer Kolben (29, 40, 50) verbunden ist, der eine kraftvervielfachende Rolle spielt und auf den betreffenden beweglichen Ring-Sitz unter Zwischenschaltung eines zweiten, vorzugsweise nicht komprimierbaren Fluids, einwirkt.

10. Vorrichtung nach Anspruch 9, dadurch gekennzeichnet, daß an dem am weitesten vom proximalen Gelenkkopf (108a) entfernten Ende des beweglichen Ring-Sitzes (25) des proximalen Gelenkkopfs (108a) ein zweiter, nicht mündender Zylinder gebildet ist, der dazu bestimmt ist, den Kopf (29') und einen Teil der Stange (29'') des sekundären Kolbens (29) aufzunehmen, der mit diesem Ring (25) verbunden ist und dessen distales Ende die proximale Wand der mit Druckluft gespeisten Arbeitskammer (27) dieses Rings (25) bildet, wobei der Raum (20), der zwischen diesem Ring (25) und dem Boden des Zylinders (106), der ihn aufnimmt, gebildet ist und der die Stange (29'') dieses sekundären Kolbens (29) umgibt, mit sekundärem Fluid gefüllt ist.

11. Vorrichtung nach einem der Ansprüche 9 und 10, dadurch gekennzeichnet, daß der Hilfskolben (47), der mit dem Zwischen-Gelenkkopf (108) verbunden ist, eine Stange (47a) aufweist, die in einer axialen Bohrung (40a) eines ersten sekundären Kolbens (40) geführt wird, dessen Stirnseite (40b) einen ringförmigen Teil des Bodens der Arbeitskammer (34) bildet, der mit dem beweglichen Ring-Sitz (36) dieses Zwischen-Gelenkkopfs (108) verbunden ist, und dessen Stange (40c) in einer axialen Bohrung (50'a) einer Stirnwand (50a) eines zweiten sekundären Kolbens (50) geführt wird, die mit einem zylindrischen Hemd (50b) versehen ist, dessen freies Ende (50'b) gegen eine ringförmige Fläche (36b) des beweglichen Ring-Sitzes (36) dieses Zwischen-Gelenkkopfes (108) drückt, die in die zu diesem Gelenkkopf entgegengesetzte Richtung gerichtet ist, wobei die Kammer (60), die zwischen dem Kopf des Hilfskolbens (47) und der Stirnseite (50a) des zweiten sekundären Kolbens (50) gebildet ist und die Stange (47a) des Hilfskolbens (47) umgibt, mit sekundärem Fluid gefüllt ist.

12. Vorrichtung nach einem der Ansprüche 9 bis 11, dadurch gekennzeichnet, daß das sekundäre Fluid Hydrauliköl ist.
